# EUROPEAN PATENT APPLICATION

(11) **EP 4 778 524 A1**
(43) Date of publication of application: **22.07.2026**
(21) Application number: 25305061.1
(22) Date of filing: 17.01.2025
(51) Int. Cl.: A61K 31/5415, A61K 31/135, A61K 31/138, A61K 31/4525, A61K 35/00, A61P 25/00

(54) **SULFOXYLATED PHENOTHIAZINES TO TREAT PAIN**

(71) Applicant: UNIVERSITE DE BORDEAUX, 33000 Bordeaux (FR); Centre National de la Recherche Scientifique, 75016 Paris (FR); Institut Polytechnique de Bordeaux, 33400 Talence (FR)
(72) Inventor: FOSSAT, Pascal, 33600 PESSAC (FR); PEIXOTO, Philippe, 33610 CESTAS (FR)
(74) Representative: Novagraaf Technologies

(57) **Abstract**

The present invention relates to sulfoxylated phenothiazine for use as a medicament.

The present invention also relates to sulfoxylated phenothiazine for use in the treatment of painful sensations induced by neuropathy.

The present invention also relates to a pharmaceutical composition comprising at least one sulfoxylated phenothiazine.

## Description

### Technical field

The present invention refers to an active agent for use as a medicament, in particular in the treatment of painful sensations induced by neuropathy.

Therefore, the present invention has utility in in the medical field.

In the description below, the references into brackets ([ ]) refer to the listing of references situated at the end of the text.

### Background of the Invention

Pain is a warning system that protects organisms against real or potential tissue damages. This unpleasant feeling can be efficiently filtered in case of excessive pain thanks to the existence of an endogenous system that control pain transmission. This endogenous system is activated by diffuse noxious inhibitory control (DNIC) also called conditioned pain modulation (CPM) in human. Deficit in CPM is a good predictor of poor pain outcome after surgery, and growing evidence link abnormal CPM to chronic pain states in patients with postoperative, neuropathic or idiopathic pain.

In this context, CPM paradigms are used to evaluate pain status. Alterations in CPM appear to result from an imbalance between excitatory and inhibitory descending pain pathways including monoaminergic nuclei (ie, serotonin and noradrenaline) of the brainstem that project to the dorsal horn of the spinal cord (DH). So far, underlying neural circuits remain largely unknown.

Therefore, the treatment of chronic neuropathic pain remains an important area of research. Indeed, current treatments have a very modest efficacy. Among these treatments, the first line is composed of tricyclic antidepressants (TCAs) and non-specific serotonin and noradrenaline reuptake inhibitors (SNRIs). Unfortunately, these molecules alone provide interesting but still very insufficient results. Indeed, the latest data from the International Association for the Study of Pain (IASP) NeuPSIG (International Neuropathic Pain Expert Group) show that the number of treatments needed to halve the pain in at least one patient is 3.6 for tricyclic antidepressants and 6.6 for SNRIs (NNT). These results were obtained after meta-analysis of 229 placebo-controlled studies.

On the other hand, these use of SNRIs leads to significant side effects and the number needed to stop treatment for adverse side effects (NNH) was 13.4 with tricyclics and 11.8 with SNRIs (data published by Finnnerup et al. ([1])).

At the same time, studies with molecules specifically targeting serotonin (SSRIs) have little or no effect on the level of pain in neuropathic patients but have fewer adverse effects.

However, a combination of a serotonin reuptake inhibitor and a KCC2-type chlorine activator has been shown to improve pain symptoms (WO2022/268738 ([2]).

Among KCC2 activators, molecules from the piperazine phenothiazine family have been studied recently (Liabeuf et al., Journal of Neurotrauma, 2017 ([3])). Prochlorperazine (PCPZ) has been identified as being able to potentiate KCC2, and to be effective in pathologies associated with this transporter (Liabeuf et al. ([3]); Donneger et al. ([4]); C. Sahara et al. ([5])). Unfortunately, PCPZ is not effective in models of chronic neuropathic pain.

Thus, a need exists of alternative efficient and well-tolerated tools for treating chronic neuropathic pain.

### Description of the invention

Thanks to extensive research work, the inventors has found surprisingly that sulfoxidation of phenothiazines, and especially of piperazine derivatives of phenothiazine, can be used to treat chronic neuropathic pain conditions.

In particular, the inventors showed that a sulfoxide form of phenothiazine, and especially PCPZ, improves painful symptoms in an animal model by restoring the animal's mechanical response thresholds, whereas PCPZ has no effect.

Accordingly, in a first aspect, the present invention provides sulfoxylated phenothiazine, or a pharmaceutically acceptable salt thereof, for use as a medicament. In other words, the present invention relates to the use of sulfoxylated phenothiazine for the manufacture of a medicament.

In another aspect, the present invention provides sulfoxylated phenothiazine, or a pharmaceutically acceptable salt thereof, for use in the treatment of painful sensations induced by neuropathy. In other words, the present invention relates to the use of sulfoxylated phenothiazine for the manufacture of a medicament for the treatment of painful sensations induced by neuropathy. Advantageously, the neuropathy is a peripheral neuropathy.

"Sulfoxylated phenothiazine" refers herein to phenothiazine or any derivative thereof, in which the sulfur atom in the phenothiazine core structure, i.e. a tricyclic compound with a structure consisting of two benzene rings linked by a sulfur atom and a nitrogen atom, has been oxidized to form a sulfoxide group (-SO).

In an embodiment of the invention, the sulfoxylated phenothiazine may be unsubstituted on its two aromatic rings. Such an unsubstituted sulfoxylated phenothiazine may be for example phenothiazine sulfoxide, also named phenothiazine-5-oxide, CAS. No. 1207-71-2, of formula (I):

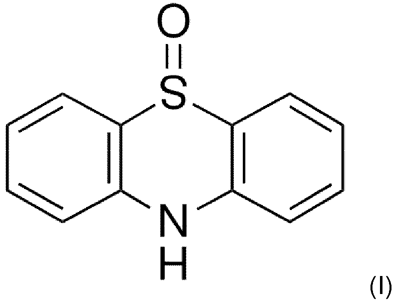

In another embodiment of the invention, the sulfoxylated phenothiazine may be monosubstituted on one of its aromatic rings, for example by a halogen, such as a chlorine atom, a fluorine atom, a bromine atom or a iodine atom, a cyano motif, an halogenoalkane, such as a trifluoromethyl motif, a methoxy motif, a methylsulfide motif, methyl sulfoxide motif, or a dimethylsulfonamide motif.

Whether the sulfoxylated phenothiazine is substituted or not on the aromatic ring(s), it may be N-substituted by an aliphatic side chain.

"Aliphatic side chain" refers to a side chain that consists solely of carbon and hydrogen atoms connected by single, double, or triple bonds, forming a linear or branched structure, excluding aromatic rings. Advantageously, aliphatic side chains may comprise 1 to 6 carbon atoms. For example, linear aliphatic side chain may be a linear alkyl selected among methyl, ethyl, n-propyl, n- butyl, n-pentyl and n-hexyl. For example, branched aliphatic side chain may be a branched alkyl selected among isopropyl, isobutyl, sec-butyl, tert-butyl, isopentyl, neopentyl, isohexyl, and neohexyl.

The aliphatic side chain attached to the nitrogen atom of the phenothiazine may possibly bear a piperazine motif, a piperidine motif, or a dimethylamine motif.

"Piperazine motif" refers herein to a chemical structure based on the piperazine ring, which is a six-membered ring containing two nitrogen atoms at opposite positions (1,4-diazacyclohexane). Piperazine motif may be N-substituted on the nitrogen atom which is opposite to the nitrogen linked to the aliphatic chain. For example, the phenothiazine N-substituted by an aliphatic side chain bearing a piperazine motif is selected in the group comprising :
- prochlorperazine, for example available as dimaleate salt (CAS [84-02-6]). Prochlorperazine sulfoxide is commercially available, for example as CAS [10078-27-0] from Santa Cruz or Merck, or as maleate salt (CAS [124552-34-7]).
- fluphenazine (CAS [63-23-8]). Sulfoxide fluphenazine is commercially available, for example as CAS [1674-76-6] from Merck or Santa Cruz, or as dihydrochloride salt as CAS [32224-60-5] from LGC Standards or Benchem.
- perphenazine (CAS [58-39-9]). Sulfoxide perphenazine is commercially available, for example as CAS [10078-25-8] from Merck or Santa Cruz Biotechnology, or as maleate [121677-10-9].
- trifluoperazine (CAS [117-89-5]). Sulfoxide trifluoperazine is commercially available, for example as CAS [1549-88-8]) from CymitQuimica or Veeprho.
- Pipotiazine (CAS [39860-99-6]). Sulfoxide pipotiazine is commercially available, for example as CAS [85275-55-4] from Chem960.

"Piperidine motif" refers herein to a chemical structure based on the piperidine ring, which is a six-membered saturated heterocyclic ring containing one nitrogen atom. Piperidine motif may be N substituted, and/or it may comprise a functional group on at least one of the carbon atom of the heterocycle. For example, the phenothiazine N-substituted by an aliphatic side chain bearing a piperidine motif is selected in the group comprising :
- periciazine (CAS [2622-26-6]). Sulfoxide periciazine may be synthesized for example as described in Blazheyevskiy and Moroz, 2019 ([6]).
- thioridazine (CAS [50-52-2]). Sulfoxide thioridazine is commercially available, for example as CAS [7776-05-8] from Santa Cruz or LGC Standards,
- mesoridazine (CAS [5588-33-0]). Sulfoxide mesoridazine is commercially available, for example as CAS [53926-89-9] from LGC Standards or from Veerpho.

"Dimethylamine motif" refers herein to a chemical motif with the formula (CH₃)₂NH, i.e. consisting of a nitrogen atom bonded to two methyl groups and one hydrogen atom, forming a tertiary amine. For example, the phenothiazine N-substituted by an aliphatic side chain bearing a dimethylamine motif is selected in the group comprising:
- chlorpromazine (CAS [50-53-3]). Chlorpromazine sulfoxide is commercially available, for example as CAS [969-99-3] from Merck or LGC Standards.
- Promazine (CAS [58-40-2]). Promazine sulfoxide is commercially available, for example as CAS [146-21-4], from LGC Standards or Simson Pharma Limited.
- Triflupromazine (CAS [146-54-3]). Triflupromazine sulfoxide is commercially available, for example as CAS [4248-38-8], from LGC, Actinopeptide, Alfa chemistry, ASW Medchem, Aurora Buiding block, or Ambinter.
- Cyamemazine (CAS [3546-03-0]. Cyamemazine sulfoxide is commercially available, for example as CAS [13384-45-7], from LGC Standards or Veeprho.
- Levomepromazine (CAS [60-99-1]). Levomepromazine sulfoxide is commercially available, for example as CAS [7606-29-3], from Merck or Santa Cruz Biotechnology.

"Pharmaceutically acceptable salts" may include inorganic as well as organic acids salts. Representative examples of suitable inorganic acids include hydrochloric (or chlorhydric), hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. The pharmaceutically acceptable salts of the compounds of the invention also include polysalts, for instance, polyhydrochlorate such as dihydrochlorate, trihydrochlorate, polymesylate such as di or tri- trimesylate, polymaleate such as di or tri-maleate. In a preferred embodiment, the salt is selected from the group consisting of hydrochloride, maleate, and methanesulfonate (or mesylate). In a more preferred embodiment, the salt is selected from the group consisting of dihydrochloride, dimesylate, dimaleate, and dioxalate.

By "treatment" is meant the curative treatment of painful sensations induced by neuropathy. A curative treatment is defined as a treatment that eases, improves and/or eliminates, reduces and/or stabilizes painful sensations induced by neuropathy. It is understood that the administered dose of sulfoxylated phenothiazine may be adapted by those skilled in the art according to the patient, the degree of the pathology, the mode of administration, etc. For instance, the compounds of the invention may be used at a dose of 0.0001 to 500 mg / day for a human patient, for example between about 40 mg and 150 mg / day, in one or more doses per day.

"Painful sensations induced by neuropathy" refers herein to forms of neuropathic pain, which result from damage, dysfunction, or irritation of the nervous system. Neuropathy can affect peripheral nerves, central nerves, i.e. in the brain or spinal cord, or a combination of both, leading to abnormal pain signaling. The treatment may have an effect on at least one of sensations comprising burning, shooting, stabbing or piercing, tingling or Paresthesia, allodynia, hyperalgesia, throbbing, numbness with Pain and deep aching pain. This pain may be chronic and differs from nociceptive pain caused by tissue injury. Advantageously, the neuropathy concerned is a peripheral neuropathy.

The sulfoxylated phenothiazines may be obtained by the chemical oxidation of the sulfur atom, for example using a procedure such as the one reported by Owens et al. ([7]) in presence of nitric acid.

Another aspect of the invention relates to a pharmaceutical composition comprising at least one sulfoxylated phenothiazine as defined above.

Sulfoxylated phenothiazine may be formulated in a pharmaceutical composition in accordance with standard pharmaceutical practice for use in a therapeutic treatment in mammals including humans. Therefore, pharmaceutical compositions of the present invention may comprise a pharmaceutically acceptable excipient.

The phrase "pharmaceutically acceptable excipient " indicates that the substance or composition must be compatible chemically and/or toxicologically, with the other ingredients of the formulation, and the mammal being treated therewith. Suitable carriers, diluents and excipients are well known to those skilled in the art and include materials such as carbohydrates, waxes, water soluble and/or swellable polymers, hydrophilic or hydrophobic materials, gelatin, oils, solvents, water and the like. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, saline, cyclodextrin, ethanol, propylene glycol, polyethylene glycols (e.g., PEG 400, PEG 300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents and flavoring agents.

The pharmaceutical compositions of the invention may be prepared using conventional dissolution and mixing procedures. For example, the bulk drug substance may be dissolved in a suitable solvent in the presence of one or more of the excipients described above.

The pharmaceutical compositions of the invention may be administered by any route appropriate to the condition to be treated, which includes oral and parenteral routes, the latter including intraperitoneal, subcutaneous, intramuscular, intravenous, intraarterial, intradermal, intrathecal, epidural, and infusion techniques. It will be appreciated that the preferred route may vary with for example the condition of the recipient. Where the composition is administered orally, it may be formulated as tablets, pills, hard or soft e.g., gelatin capsules, cachets, troches, lozenges, aqueous or oil suspensions, dispersible powders or granules, emulsions, syrups or elixirs each containing a predetermined amount of each active agent, with a pharmaceutically acceptable carrier, glidant, or excipient. Where the composition is administered parenterally, it may be formulated with a pharmaceutically acceptable parenteral vehicle or diluent, and in a unit dosage injectable form, as detailed below. For example, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

Advantageously, the pharmaceutical composition may comprise at least one other active ingredient, i.e. an active ingredient different than a sulfoxylated phenothiazine. For example, the other ingredient may be at least one Serotonin reuptake inhibitors (SRI), which are antidepressant. Examples of SRI are given in the documents "NICE Clinical Guidelines, No. 31. National Collaborating Centre for Mental Health (UK). Leicester (UK): British Psychological Society (UK); 2006" ([8]); Gründer et al. ([9]); Moses-Kolko et al. ([10]), Hughes et al. ([11]), Bartoszyk et al. ([12]) et Gerald P. Koocher et al. ([13]). Advantageously, SRI may be selected among serotonin specific reuptake inhibitors (SSRI), serotonin noradrenaline reuptake inhibitors (SNRI) and tricyclic antidepressants (TCA). Advantageously, SSRI may be selected among fluoxetine, paroxetine, sertraline, citalopram, escitalopram oxalate, indalpine, zimelidine, vilazadone, dapoxetine, vortioxetine and fluvoxamine maleate, SNRI may be selected among duloxetine, desvenlafaxine, levomilnacipran, milnacipran and venlafaxine, and TCA may be selected among amitriptyline, amoxapine, desipramine, doxepin, imipramine, nortriptyline, protriptyline and trimipramine. Preferably, SSRI may be fluoxetine or paroxetine, SNRI may be duloxetine and TCA may be amitriptyline. According to the invention, "at least one" refers to 1, or 2, or 3, or 4, or more of other active agent(s).

Sulfoxylated phenothiazine, especially when used in the form of a pharmaceutical composition, may be administered to a subject in need thereof at a therapeutically effective amount. The phrase "therapeutically effective amount" means an amount of a compound of the present invention that (i) treats the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In case of the treatment of painful sensations induced by peripheral neuropathy, the therapeutically effective amount of sulfoxylated phenothiazine may reduce mechanical allodynia of about 50% 24h after injection and still about 25% 48hrs after injection.

As an example, a pharmaceutically effective amount of sulfoxylated phenothiazine administered per dose may be in the range of about 0.1-10mg/kg, with a maximum of 40 mg / day, depending on the route of administration, with the typical initial range of compound used being 20 to 40 mg/day (i.e. for an adult of 70kg a doses of approximately 0.6 mg/kg/day).

As an example, a pharmaceutically effective amount of SSRI, SNRI and/or TCA administered per dose may be in the range of about 0.1-10mg/kg, with a maximum of 40 mg / day, depending on the route of administration, with the typical initial range of compound used being 20 to 40 mg/day (i.e. for an adult of 70kg a doses of approx. 0.6mg/kg/day).

This invention is further illustrated by the following examples with regard to the annexed drawings that should not be construed as limiting.

### Brief description of the figures

- Figure 1: represents the synthesis of prochlorperazine sulfoxide, as detailed in Owens et al. ([7]).
- Figure 2: represents the mechanical response threshold (g) as a function of the time (days) of drug injection (PCPZ 2mg/kg (circles) or PCPZ sulfoxide 2 mg/kg (squares)), i.e. before any nerve damage, after nerve damage, and then after this time, 1 to 3 days after injection of the drugs.

### Examples

### Example 1: preparation of a sulfoxydated form of PCPZ and its maleate salt.

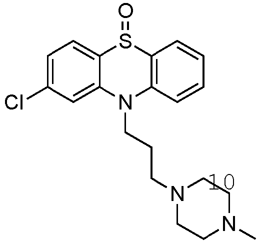

Using a procedure adapted from Owens et al. ([7]). Commercially available prochlorperazine dimaleate (150 mg, 0.25 mmol, 1 equiv.) was added to a saturated aqueous solution of Na₂CO₃ (5 mL). After 10 min at room temperature, this solution was extracted 3 times with ethyl acetate (3 x 5 mL). The combined organic layers were dried over anhydrous Na₂SO₄, filtered, and concentrated under reduced pressure to provide salt-free prochlorperazine as an colorless oil (95 mg, 0,25 mmol).

To a stirred aqueous solution (5 mL) containing 95 mg of salt-free prochlorperazine (0.25 mmol, 1 equiv.) was added 0,24 mL of a 37% aqueous hydrogen chloride solution (2,8 mmol, 11,2 equiv.), followed by 2,67 mL of a 10% aqueous NaNO₂ solution (3.9 mmol, 15,5 equiv.). Argon was then bubbled through the resulting solution to remove nitrogen oxide gases while the reaction mixture was stirred for 45 min. The reaction was then quenched with a 28% aqueous ammonium hydroxide solution (5 mL), and extracted trice with ethyl acetate (3 x 10 mL). The combined organic layers were dried over anhydrous Na₂SO₄, filtered, and concentrated under reduced pressure. The crude product was purified by silica gel chromatography, eluting with a mixture of CH₂Cl₂/MeOH/acetone (9/1/1), to afford the prochlorperazine sulfoxide as a white solid (87 mg, 89%). **R*f*** = 0.08 (CH₂Cl₂/MeOH - 9/1)

**1H NMR** (300 MHz, CDCl₃) δ = 7.87 (dd, *J* = 7.7, 1.6 Hz, 1H), 7.78 (d, *J =* 8.2 Hz, 1H), 7.66 (m, 1H), 7.57 (m, 2H), 7.33-7,27 (m, 1H), 7.23 (dd, *J =* 8,3, 1,8 Hz, 1H), 4.31 - 4.22 (t, 2H), 2.60 - 2.32 (m, 10H), 2.27 (s, 3H), 1.99 (m, 2H).

**13C NMR** (75 MHz, CDCl₃) δ 139.4, 138.9, 137.8, 133.0, 132.8, 131.7, 124.4, 122.7, 122.3, 121.9, 116.2, 116.1, 55.2 (2C), 54.7, 53.4 (2C), 46.1, 45.6, 23.8.

**HRMS (ESI)** exact mass calculated for C₂₀H₂₄ClN₃OS: m/z 390.1401 ([M+H]⁺), found: m/z 390.1389 ([M+H]⁺).

**IR** *ν*ₘₐₓ = 2937; 2795; 1580; 1547; 1455; 1421; 1352; 1279; 1246; 1147; 1029; 919; 751; 494; 465; 455 cm⁻¹. 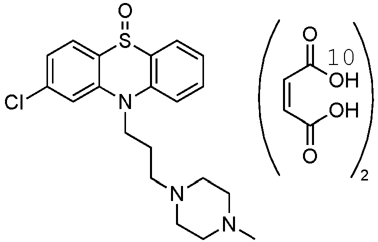 Prochlorperazine sulfoxide (51.4 mg, 0.131 mmol) was added 0,5 mL of EtOH, and further dissolved by heating the reaction mixture for 2 min at 78°C. To this stirred solution was added 30 mg of maleic acid (0.262 mmol, 2 equiv.) and 2,5 mL of EtOH (2.5 ml). The reaction mixture was then stirred for 10 min at 78°C until a precipitation of the salt occurred. The reaction mixture was then reduced under pressure to afford to desired water soluble prochlorperazine dimaleate as an off-white solid (82 mg, 0,131 mmol) in a quantitative yield.

**1H NMR** (300 MHz, D₂O) δ 7.93 (dd, J = 7.7, 1.5 Hz, 1H), 7.83 (m, 1H), 7.77 (d, *J* = 8.3 Hz, 1H), 7.63 (d, *J* = 8.5 Hz, 1H), 7.56 (d, *J* = 1.7 Hz, 1H), 7.43 (t, *J* = 7,5 Hz, 1H), 7.31 (dd, *J* = 8.3, 1.8 Hz, 1H), 6.30 (s, 4H), 4.46 - 4.28 (m, 2H), 3.47 (brs, 4H), 3,30 (brs, 4H), 3.02 (dd, *J* = 9.4, 6.9 Hz, 2H), 2,90 (s, 3H), 2.20 (m, 2H).

**IR** *ν*ₘₐₓ = 3441; 2924; 1701; 1625; 1580; 1461; 1385; 1356; 1309; 1252; 1107; 1054; 1020; 942; 865; 804; 754; 647; 586; 475 cm⁻¹.

### Example 2: Effect of PCPZ sulfoxide on mechanic hypersensibility

The PCPZ sulfoxide made in Example 1 was tested in a mouse model of peripheral neuropathy caused by nerve damage: a nerve lesion induces mechanical hypersensitivity resulting in a drop in the force required to withdraw the animal's paw (Aby et al., 2022 ([14]), Decosterd et al., 2000 ([15]). Mechanical response threshold (g) was measured as a function of the time of drug injection (PCPZ 2mg/kg or PCPZ sulfoxide 2 mg/kg), i.e. before any nerve damage, 15 days after nerve damage, and then after this time, 1 to 3 days after injection of the drug.

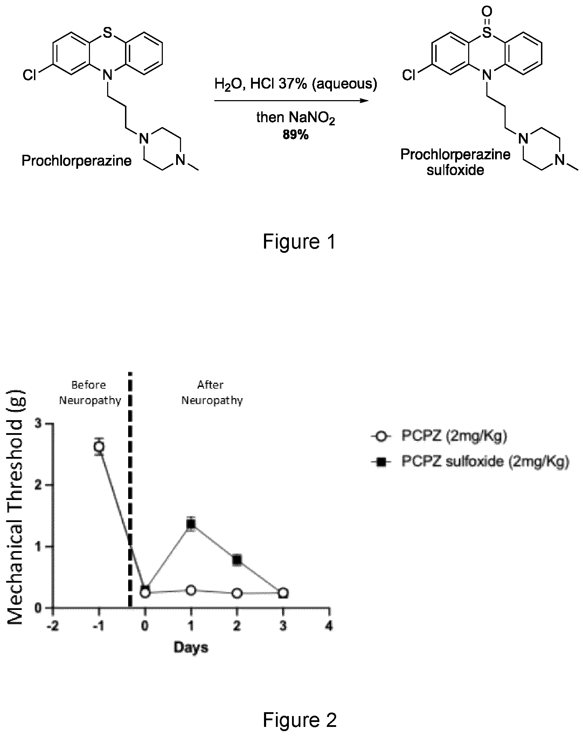

As shown in Figure 2, PCPZ sulfoxide attenuates mechanical hypersensitivity in the animal model. With PCPZ sulfoxide, the thresholds increase again (squares) whereas they are not modified with PCPZ non sulfoxydated (circles).

Therefore, these results show that sulfoxydated PCPZ improves pain symptoms by restoring the animal's mechanical response thresholds, whereas non sulfoxydated form of PCPZ has no effect.

In conclusion, sulfoxydated phenothazines can be used to treat chronic neuropathic pain.

### Reference List

1. Finnerup, Nanna B et al. Pharmacotherapy for neuropathic pain in adults: a systematic review and meta-analysis. The Lancet Neurology, Volume 14, Issue 2, 162 - 173, February 2014.
2. WO2022/268738.
3. Liabeuf S, Stuhl-Gourmand L, Gackière F, Mancuso R, Sanchez Brualla I, Marino P, Brocard F, Vinay L. Prochlorperazine Increases KCC2 Function and Reduces Spasticity after Spinal Cord Injury. J Neurotrauma. 2017 Dec 15;34(24):3397-3406. doi: 10.1089/neu.2017.5152. Epub 2017 Sep 19. PMID: 28747093.
4. Florian Donneger, Adrien Zanin, Jeremy Besson, Yoness Kadiri, Carla Pagan, Nicolas David, Marion Russeau, Franck Bielle, Bertrand Devaux, Bertrand Mathon, Vincent Navarro, Francine Chassoux, Jean Christophe Poncer. Enhancing KCC2 function reduces interictal activity and prevents seizures in mesial temporal lobe epilepsy. bioRxiv 2023.09.16.557753; doi: https://doi.org/10.1101/2023.09.16.557753.
5. C. Sahara Khademullah, Julien Bourbonnais, Mathilde M. Chaineau, Maria José Castellanos-Montiel, lason Keramidis, Alexandra Legault, Marie-Ève Paquet, Agessandro Abrahao, Lorne Zinman, Janice Robertson, Thomas M. Durcan, Melanie A. Woodin, Antoine G. Godin, Yves De Koninck. KCC2 as a novel biomarker and therapeutic target for motoneuron degenerative disease. bioRxiv 2023.08.24.554410; doi: https://doi.org/10.1101/2023.08.24.554410.
6. Blazheyevskiy and Moroz. A new oxidative derivatization method for spectrophotometric determination of Periciazine in pharmaceutical preparations. French-Ukrainian journal of chemistry (2019, volume 07, issue 02).
7. Owens ML, Juenge EC, Poklis A. Convenient oxidation of phenothiazine salts to their sulfoxides with aqueous nitrous acid. J Pharm Sci. 1989 Apr;78(4):334-6.
8. NICE Clinical Guidelines, No. 31. National Collaborating Centre for Mental Health (UK). Leicester (UK): British Psychological Society (UK); 2006.
9. Gründer G, Hiemke C, Paulzen M, Veselinovic T, Vernaleken I (2011). "Therapeutic plasma concentrations of antidepressants and antipsychotics: lessons from PET imaging". Pharmacopsychiatry. 44 (6): 236-48.
10.Moses-Kolko EL, Bogen D, Perel J, et al. Neonatal Signs After Late In Utero Exposure to Serotonin Reuptake Inhibitors: Literature Review and Implications for Clinical Applications. JAMA. 2005;293(19):2372-2383.
11.Hughes ZA, Starr KR, Langmead CJ, et al. Neurochemical evaluation of the novel 5-HT1A receptor partial agonist/serotonin reuptake inhibitor, vilazodone. Eur J Pharmacol 2005;510(1-2):49-5.
12.Bartoszyk, R. Hegenbart, H. Ziegler EMD 68843, a serotonin reuptake inhibitor with selective presynaptic 5-HT1A receptor agonistic properties Eur. J. Pharmacol., 322 (1997), pp. 147-153.
13.Gerald P. Koocher; John C. Norcross; Beverly A. Greene (2013). Psychologists' Desk Reference. Oxford University Press. pp. 407. ISBN 978-0-19-984550-7.
14. Franck Aby, Louis-Etienne Lorenzo, Zoé Grivet, Rabia Bouali-Benazzouz, Hugo Martin, Stéphane Valerio, Sara Whitestone, Dominique Isabel, Walid Idi, Otmane Bouchatta, Philippe De Deurwaerdere, Antoine G. Godin, Cyril Herry, Xavier Fioramonti, Marc Landry, Yves De Koninck, and Pascal Fossat, Switch of serotonergic descending inhibition into facilitation by a spinal chloride imbalance in neuropathic pain, Science Advances, 27 Jul 2022, Vol 8, Issue 30, DOI: 10.1126/sciadv.abo0689.
15.Isabelle Decosterd, Clifford J Woolf, Spared nerve injury: an animal model of persistent peripheral neuropathic pain, Pain, Volume 87, Issue 2, 2000, Pages 149-158, ISSN 0304-3959, https://doi.org/10.1016/S0304-3959(00)00276-1.

## Claims

1. Sulfoxylated phenothiazine for use as a medicament.

2. Sulfoxylated phenothiazine for use in the treatment of painful sensations induced by neuropathy.

3. Sulfoxylated phenothiazine for use according to claim 2, wherein said neuropathy is a peripheral neuropathy.

4. Sulfoxylated phenothiazine for use according to any one of the preceding claims, wherein said phenothiazine is N-substituted by an aliphatic side chain, possibly bearing a piperazine motif, a piperidine motif, or a dimethylamine motif.

5. Sulfoxylated phenothiazine for use according to any one of the preceding claims, wherein said phenothiazine is unsubstituted on aromatic rings, or monosubstituted on one of aromatic rings by a chlorine atom, a cyano motif, a trifluoromethyl motif, a methoxy motif, a methylsulfide motif, methyl sulfoxide motif, or a dimethylsulfonamide motif.

6. Sulfoxylated phenothiazine for use according to claim 4, wherein said phenothiazine N-substituted by an aliphatic side chain bearing a piperazine motif is selected in the group comprising prochlorperazine, fluphenazine, perphenazine, trifluoperazine, and pipotiazine.

7. Sulfoxylated phenothiazine for use according to claim 4, wherein said phenothiazine N-substituted by an aliphatic side chain bearing a dimethylamine motif is selected in the group comprising chlorpromazine, promazine, triflupromazine, cyamemazine, and levomepromazine.

8. Sulfoxylated phenothiazine for use according to claim 4, wherein said phenothiazine N-substituted by an aliphatic side chain bearing a piperidine motif is selected in the group comprising periciazine, thioridazine and mesoridazine.

9. Pharmaceutical composition comprising at least one sulfoxylated phenothiazine as defined in any one of claims 1 to 8.

10. Pharmaceutical composition according to claim 9, which is in a oral or parenteral form, for example intraperitoneal form.

11. Pharmaceutical composition according to claim 9 or 10, which comprises a pharmaceutically acceptable excipient.

12. Pharmaceutical composition according to any one of claims 9 to 11, comprising at least one other active ingredient.

13. Pharmaceutical composition according to claim 12, wherein the at least one other ingredient is selected among serotonin specific reuptake inhibitors (SSRI), serotonin noradrenaline reuptake inhibitors (SNRI) and tricyclic antidepressants (TCA).

14. Pharmaceutical composition according to claim 13, wherein SSRI is selected among fluoxetine, paroxetine, sertraline, citalopram, escitalopram oxalate, indalpine, zimelidine, vilazadone, dapoxetine, vortioxetine and fluvoxamine maleate, SNRI is selected among duloxetine, desvenlafaxine, levomilnacipran, milnacipran and venlafaxine, and TCA is selected among amitriptyline, amoxapine, desipramine, doxepin, imipramine, nortriptyline, protriptyline and trimipramine.

15. Pharmaceutical composition according to claim 14, wherein SSRI is fluoxetine or paroxetine, SNRI is duloxetine, and TCA is amitriptyline.
